# EUROPÄISCHE PATENTSCHRIFT

(11) **EP 3 596 474 B1**
(45) Veröffentlichungstag und Bekanntmachung des Hinweises auf die Patenterteilung: **17.02.2021**
(21) Anmeldenummer: 17835797.6
(22) Anmeldetag: 05.12.2017
(51) Int. Cl.: G01N 33/68

(54) **VERFAHREN ZUR CHROMATOGRAFISCHEN QUANTITATIVEN BESTIMMUNG DER AMINOSÄUREN VALIN, METHIONIN, ALLO-ISOLEUCIN, ISOLEUCIN, LEUCIN, TYROSIN UND PHENYLALANIN IM BLUTPLASMA**
METHOD FOR THE CHROMATOGRAPHIC QUANTITATIVE DETERMINATION OF THE AMINO ACIDS VALINE, METHIONINE, ALLO-ISOLEUCINE, ISOLEUCINE, LEUCINE, TYROSINE AND PHENYLALANINE IN BLOOD PLASMA
PROCÉDÉ DE DÉTERMINATION QUANTITATIVE CHROMATOGRAPHIQUE DES ACIDES AMINÉS VALINE, MÉTHIONINE, ALLO-ISOLEUCINE, ISOLEUCINE, LEUCINE, TYROSINE ET PHÉNYLALANINE DANS LE PLASMA SANGUIN

(30) Priorität: 16.03.2017 DE 102017002523
(43) Veröffentlichungstag der Anmeldung: 22.01.2020
(73) Patentinhaber: Membrapure GmbH, 16761 Hennigsdorf (DE)
(72) Erfinder: FLINDT, Erdmann, 14193 Berlin (DE); TIMMERMANN, Detlef, 31860 Emmerthal (DE)
(74) Vertreter: Habenicht, Wieland
(86) Internationale Anmeldenummer: PCT/DE2017/000412
(87) Internationale Veröffentlichungsnummer: WO 2018/166546

(56) Entgegenhaltungen:
- DE-B3-102016 010 887
- US-A1- 2011 085 983
- PRINSEN HUBERTUS C ET AL: "Rapid quantification of underivatized amino acids in plasma by hydrophilic interaction liquid chromatography (HILIC) coupled with tandem mass-spectrometry", JOURNAL OF INHERITED METABOLIC DISEASE, KLUWER, DORDRECHT, NL, Bd. 39, Nr. 5, 21. April 2016 (2016-04-21) , Seiten 651-660, XP036033275, ISSN: 0141-8955, DOI: 10.1007/S10545-016-9935-Z [gefunden am 2016-04-21]
- YONG QU ET AL: "Quantitative amino acid analysis using a Beckman system gold HPLC 126AA analyzer", CLINICA CHIMICA ACTA, Bd. 312, Nr. 1-2, 1. Oktober 2001 (2001-10-01), Seiten 153-162, XP055457019, AMSTERDAM, NL ISSN: 0009-8981, DOI: 10.1016/S0009-8981(01)00615-5
- FREETO S M ET AL: "A rapid UPLC-MS/MS method for the determination of specific amino acids associated with maple syrup urine disease and phenylketonuria", CLINICAL CHEMISTRY, AMERICAN ASSOCIATION FOR CLINICAL CHEMISTRY, WASHINGTON, DC, Bd. 52, Nr. 6, Suppl. S, 31. Mai 2006 (2006-05-31), Seite A34, XP009503925, ISSN: 0009-9147
- PIRAUD MONIQUE ET AL: "Ion-pairing reversed-phase liquid chromatography/electrospray ionization mass spectrometric analysis of 76 underivatized amino acids of biological interest: a new tool for the diagnosis of inherited disorders of amino acid metabolism", RAPID COMMUNICATIONS IN MASS SPECTROMETRY, JOHN WILEY & SONS, GB, Bd. 19, Nr. 12, 1. Januar 2005 (2005-01-01), Seiten 1587-1602, XP002470321, ISSN: 0951-4198, DOI: 10.1002/RCM.1957

## Beschreibung

Die Erfindung betrifft ein Verfahren zur chromatografischen quantitativen Erfassung der Aminosäuren Valin, Methionin, allo-Isoleucin, Isoleucin, Leucin, Tyrosin und Phenylalanin im Blutplasma.

Nachdem die Technik der Tandem-Massenspektrometrie in der Proteomics, Metabolomics und auf der Suche nach neuen Biomarker für die Früherkennung von Krankheiten reformiert hat, wird sie heute in der klinischen Analytik routinemäßig eingesetzt. Die Tandem-Massenspektrometrie konnte das Spektrum der Diagnostik von angeborenen Stoffwechselerkrankungen (Phenylketonurie, Ahornsirupkrankheit) im Neugeborenen-Screening erweitern. Für den Screeningtest werden einige Bluttropfen mithilfe der Tandem-Massenspektrometrie auf Aminosäuren analysiert. Aufgrund ihrer identischen molekularen Massen ist eine Unterscheidung von den Aminosäuren Leucin (Leu) und Isoleucin (Ile) mittels Massenspektrometrie jedoch nicht möglich und es können falsch positive Ergebnisse entstehen. Daher wird zur Kontrolle in einer zweiten Analyse die Aminosäuren Isoleucin und Leucin mithilfe eines Aminosäureanalysator oder HPLC bestimmt. Geeignete Biomarker für die Prädiktion eines Typ2 Diabetes können die Aminosäuren Isoleucin, Leucin, Valin, Tyrosin und Phenylalanin sein. In einer im "Nature Medicine" veröffentlichten Studie (Wang T. et. al.: Metabolite profiles and the risk of developing diabetes) konnten die Wissenschaftler bereits vor Ausbruch der Krankheit Diabetes Typ 2 veränderte Konzentrationen der Aminosäuren im Blutplasma feststellen. Bei einer Homocysteinämie ist die Konzentration von der Aminosäure Homocystein im Blutplasma erhöht, und stellt ein Risikofaktor für die Entstehung von Herz-Kreislauf-Erkrankungen dar. Homocystein wird als bekannter Biomarker in der Diagnostik für Herz-Kreislauf-Erkrankungen eingesetzt. Die Bestimmung von Homocystein in Blutplasma erfolgt mithilfe eines enzymatischen Test oder HPLC.

US 2011/085983 A1 und "PRINSEN HUBERTUS C ET AL: "Rapid quantification of underivatized amino acids in plasma by hydrophilic interaction liquid chromatography (HILIC) coupled with trandom mass-spectrometry", JOURNALN OF INHERITED METABOLIC DISEASE, KLUWER, DORDRECHT, NL, Bd. 39, Nr. 5, 21. April 2016 (2016-04-21), Seiten 651-660, XP036033275, ISSN: 0141-8955, DOI: 10.1007/S10545-016-9935-Z [gefunden am 2016-04-21]", offenbaren ein Verfahren zur chromatografischen quantitativen Bestimmung der Aminosäuren Valin, Methionin, allo-Isoleucin, Isoleucin, Leucin, Tyrosin und Phenylalanin im Blutplasma, in dem die Aminosäure Homocystein in einem einzigen Chromatografielauf quantitativ miterfasst wird und bei dem es sich um ein mehrere Eluenten und ein Trennprogramm verwendendes chromatografisches Verfahren handelt, wobei als Eluate eine Kombination von TDFHA acid und TDFHA acetonitril verwendet wird, die über zwei verschiedene Säulen laufen.

"Yong Qu ET AL: "Quantitative amino acid analysis using a Beckman system gold HPLC 126AA analyzer", CLINICA CHIMICA ACTA, Bd. 312, Nr. 1-2, 1. Oktober 2001 (2001-10-01), Seiten 153-162, XP055457019, DOI 10.1016/S0009-8981(01)00615-5" und "FREETO S M ET AL: "A rapid UPLC-MS/MS method for the determination of specific amino acids associated with maple syrup urine disease and phenylketonuria", CLINICAL CHEMISTRY, AMERICAN ASSOCIATION FOR CLINICAL CHEMISTRY, WASHINGTON, DC, Bd. 52, Nr. 6, Suppl. S, 31. Mai 2006 (2006-05-31), Seite A34, XP009503925, ISSN: 0009-9147" offenbaren die Quantifizierung von 7 verschiedenen Aminosäuren, wobei Lithium basierte Puffer als Eluenten verwendet werden.

"PIRAUD MONIQUE ET AL: "Ion-pairing reversed-phase liquid chromatography/electrospray ionization mass spectrometric analysis of 76 underivatized amino acids of biological interest: a new tool fort he diagnosis of inherited disorders of amino acid metabolism", RAPID COMMUNICATIONS IN MASS SPECTROMETRY, JOHN WILEY & SONS, GB, Bd. 19, Nr. 12, 1. Januar 2005 (2005-01-01), Seiten 1587-1602, XP002470321, ISSN: 0951-4198, DOI: 10.1002/RCM.1957" offenbaren ein Verfahren zur chromatografischen quantitativen Bestimmung der Aminosäuren Valin, Methionin, allo-Isoleucin, Isoleucin, Leucin, Tyrosin und Phenylalanin.

DE 10 2016 010887 B3 offenbart ein Verfahren zum qualitativen Nachweis von zugesetztem Blutplasma in Lebensmitteln in Fleisch und Fleischwaren, wobei die Lebensmittel bei der Probenaufbereitung in Präzipitat- und Fluid-Phasen getrennt werden, wobei zumindest ein Teil der Fluidphase auf Aminosäuren qualitativ und quantitativ untersucht wird, wobei die ermittelten Probengehalte dieser Fluidphasen-Aminosäuren verglichen werden mit solchen Probegehalten, die aus den entsprechenden, nicht mit Blutplasma behandelten Lebensmitteln stammen, wobei bei der Untersuchung auf zugesetztes Blutplasma in der Fluidphase die Aminosäuren Ornithin und Cystin berücksichtigt werden.

Die Erfindung betrifft eine Methode zur Bestimmung der Konzentrationen von der Aminosäurenkombination Homocystein, Valin, Methionin, allo-Isoleucin, Isoleucin, Leucin, Tyrosin und Phenylalanin im Blutplasma. Die hier aufgeführten 8 Aminosäuren werden mit einem Analysenlauf / Chromatografielauf bestimmt. Für die Bestimmung von Homocystein wird daher kein weiterer Test benötigt, das spart Zeit und Kosten.

Es ist erfindungsgemäß, wenn es sich beim Verfahren um ein, mehrere Eluenten und ein Trennprogramm verwendendes chromatografisches Verfahren handelt.

Dies gilt auch dahingehend, dass es sich bei den Eluenten um folgende handelt, mit jeweils folgender relativer Zusammensetzung:
B: Lithiumhydroxid-Monohydrat 2,5 g, Borsäure 0,5 g, Ethanol 5 ml, Caprylsäure 0,1 ml, Trifluoressigsäure-Lösung 50% 4,7 ml, Reinstwasser 480 ml;
C: Lithiumhydroxid-Monohydrat 2,1 g, Borsäure 1,5 g, Ameisensäure 1,5 ml, Essigsäure 100% 1,5 ml, Caprylsäure 0,1 ml, Trifluoressigsäure-Lösung 50% 0,8 ml, Reinstwasser 495 ml.

In diesem Kontext ist es weiterhin vorteilhaft, wenn die Eluenten bei nachfolgenden pH-Werten verwendet werden:
Eluent B: pH-Wert entspricht 3,46 sowie Eluent C: pH-Wert entspricht 4,48.

Weiterhin ist es in diesem Kontext vorteilhaft, da bewährt, dass die Eluenten B bis C nacheinander in der Reihenfolge B, C verwendet werden.

In der Praxis hat es sich insbesondere bewährt, wenn das folgende Trennprogramm verwendet wird, wobei die Daten sich auf eine Eluentenflussrate von 240 Mikroliter je Minute und folgenden Eluenten- und Säulentemperaturverlauf beziehen:
Eluent B: Zeit ab 0 Stunden, 0 Minuten bis 9 Minuten, 57 Sekunden; Eluent C: Zeit ab 9 Minuten, 57 Sekunden bis 40 Minuten, 3 Sekunden;

Säulentemperaturverlauf:
Zeit: 0 Stunden, 0 Minuten bis 34 Minuten, 28 Sekunden;
Säulentemperatur: +57 Grad Celsius bei 0 Stunden, 0 Minuten;
Säulentemperatur: +57 Grad Celsius bei 34 Minuten, 28 Sekunden;

Zeit: 34 Minuten, 28 Sekunden bis 37 Minuten, 2 Sekunden;
Säulentemperatur: +57 Grad Celsius bei 34 Minuten, 28 Sekunden;
Säulentemperatur: +70 Grad Celsius bei 37 Minuten, 2 Sekunden;

Zeit: 37 Minuten, 2 Sekunden bis 40 Minuten, 3 Sekunden;
Säulentemperatur: +70 Grad Celsius bei 37 Minuten, 2 Sekunden;
Säulentemperatur: +70 Grad Celsius bei 40 Minuten, 3 Sekunden,
wobei zwischen den jeweiligen zeitlichen Anfangs- und Endpunkten bei angegebenen Temperaturdifferenzen der Temperaturverlauf zwischen den unterschiedlichen Temperaturniveaus im wesentlichen linear verläuft.

Aufgrund der sehr komplexen Wechselwirkung von Temperatur, pH-Wert, Dipolmoment und Polarisierbarkeit der Eluenten und deren Bestandteilen war es bisher nicht möglich, Homocystein quantitativ chromatografisch zusammen mit den oben genannten, weiteren Aminosäuren mitzuerfassen. Erfindungsgemäß ist dies nun überraschenderweise gelungen.

Die nachfolgenden Ausführungen erläutern in nicht-beschränkender Weise die Erfindung.

### Methodenentwicklung und Validierung

### Material

Eine Blutplasmaprobe wurde für die Methodenentwicklung und Validierung wie folgt aufbereitet.

### Probenaufbereitung

Das gebundene Homocystein wird mit einer 4% igen Dithiothreitollösung in freies Homocystein reduziert, hierbei wird das Homocystein aus seiner Bindung an Proteine und aus Disulfiden gelöst. Damit ist die Bestimmung des Gesamthomocystein möglich.

Probenaufbereitung: 500 µl Plasma + 100 µl 4% Dithiothreitollösung in ein Eppendorfhütchen geben, mischen, und inkubieren bei 40 °C für 30 Min., anschließend Zugabe von 150 µl 10% ige Sulfosalicylsäurelösung für die Proteinfällung im Kühlschrank 30 Min. aufbewahren.

Anschließend 500 µl Probenverdünnungspuffer (mit internen Standard Norleucin 100 nmol/ ml) zugeben und bei 14000 U/Min. 5 Min. zentrifugieren. Den Überstand abpipettieren und ein zweites Mal mit einen Membraspinfilter bei 14000 U/Min. 5 Min. zentrifugieren. Die Plasmaprobe ist fertig für die Aminosäureanalytik.

### Aminosäureanalytik

Für eine optimale Trennung und Reproduzierbarkeit wurden speziell Trennsäule, Eluenten und Trennprogramm entwickelt und eingesetzt. Die Trennsäule gepackt mit Polymerharz aus Styrol und Divinylbenzol und einer Vernetzung von 10%. Die Abmessungen der Trennsäule sind 4 mm im Innendurchmesser und 118 mm Innenlänge und 125 mm Außenlänge.

Die 2 Elueten, bezeichnet B und C, zusammengesetzt:
B: Lithiumhydroxid-Monohydrat 2,5 g, Borsäure 0,5 g, Ethanol 5 ml, Caprylsäure 0,1 ml, Trifluoressigsäure-Lsg. 50% 4,7 ml, Reinstwasser 480 ml.
C: Lithiumhydroxid-Monohydrat 2,1 g, Borsäure 1,5 g, Ameisensäure 1,5 ml, Essigsäure 100% 1,5 ml, Caprylsäure 0,1 ml, Trifluoressigsäure-Lsg. 50% 0,8 ml, Reinstwasser 495 ml.
mit unterschiedlichen pH-Wert:, Eluent B 3,46, Eluent C 4,48,

Als Regenerationslösung (Eluent F) zur Regenerierung der Trennsäule nach Trennung der Aminosäuren aus der Probe, wird eine Lithiumhydroxid-Lösung, pH-Wert 12,43 verwendet.

Der pH Wert der Eluenten wird schrittweise von Eluent B nach Eluent F erhöht (siehe Grafik 1). Hierbei ist der pH von 3,48 in der ersten Stufe am niedrigsten und erhöht sich bis zur Stufe Regeneration der Säule mit Eluent F auf 12,46.

Die Temperatur wird in mehreren Stufen geändert. Die Regelung der Säulentemperatur (siehe Tabelle 1) ist auf das Verfahren beschränkt und wird über einen Reaktor ausgeführt.

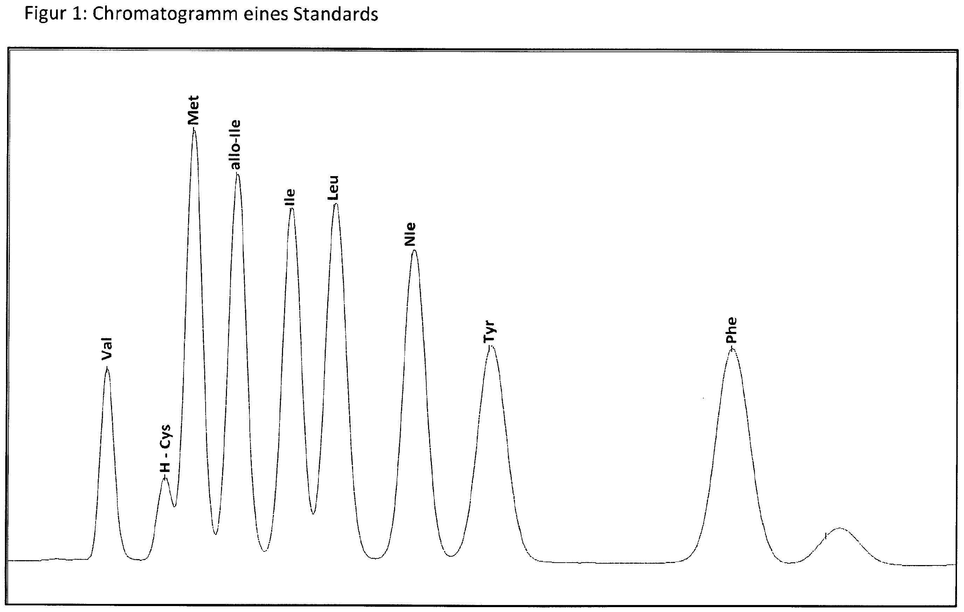

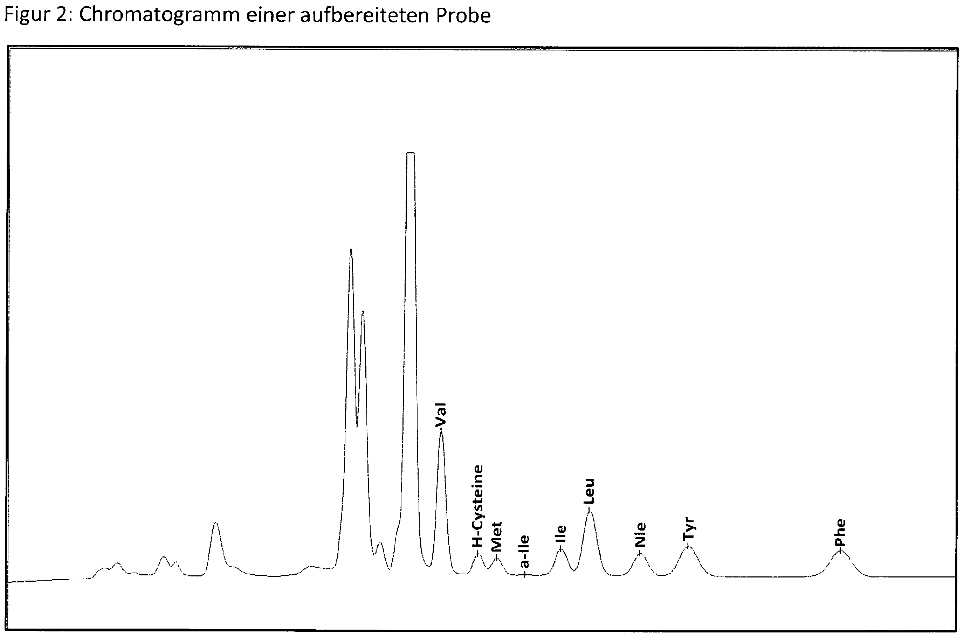

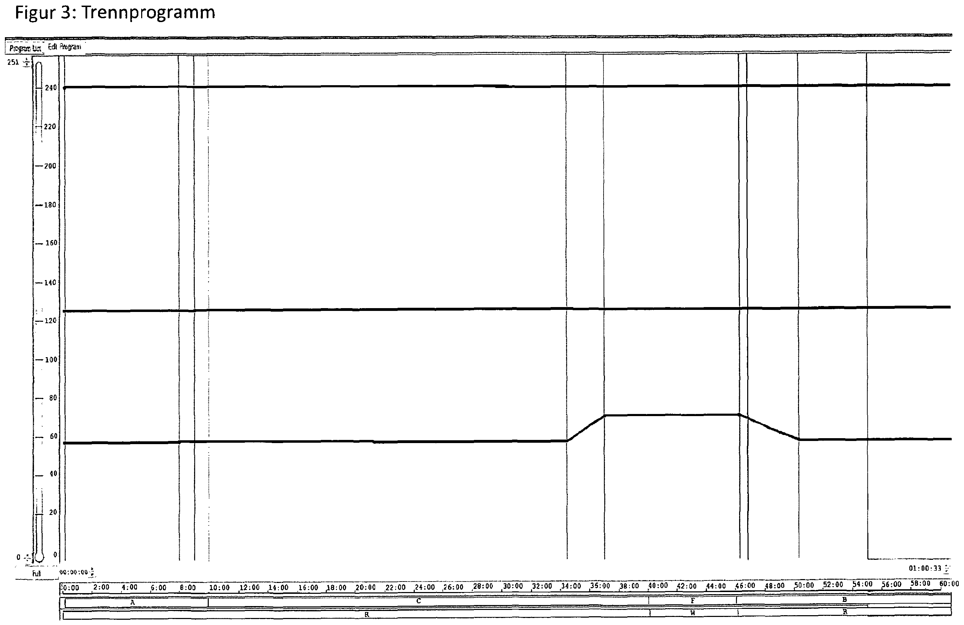

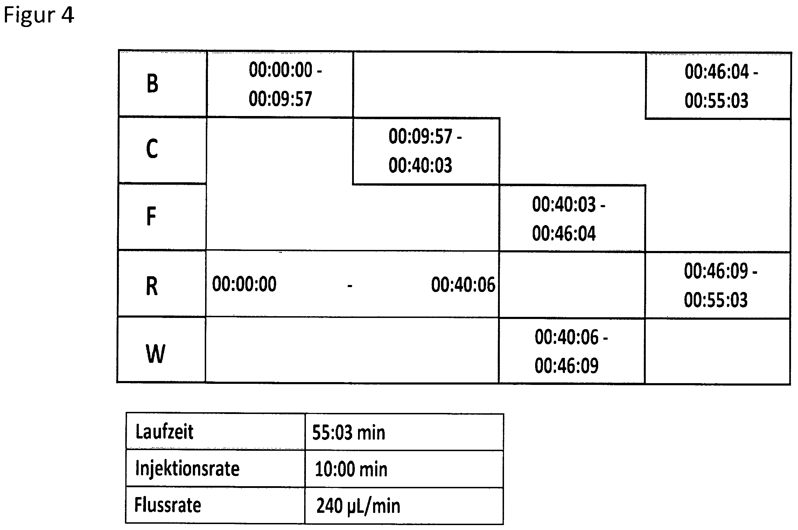

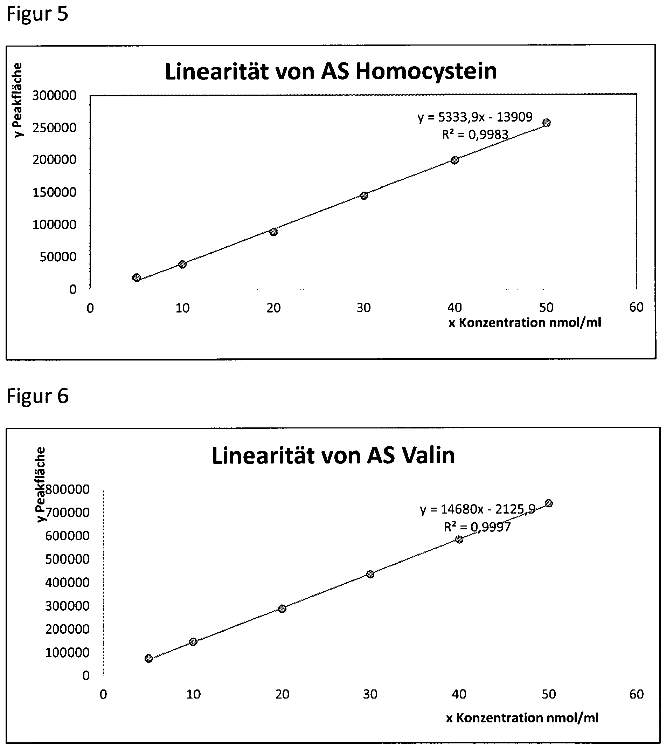

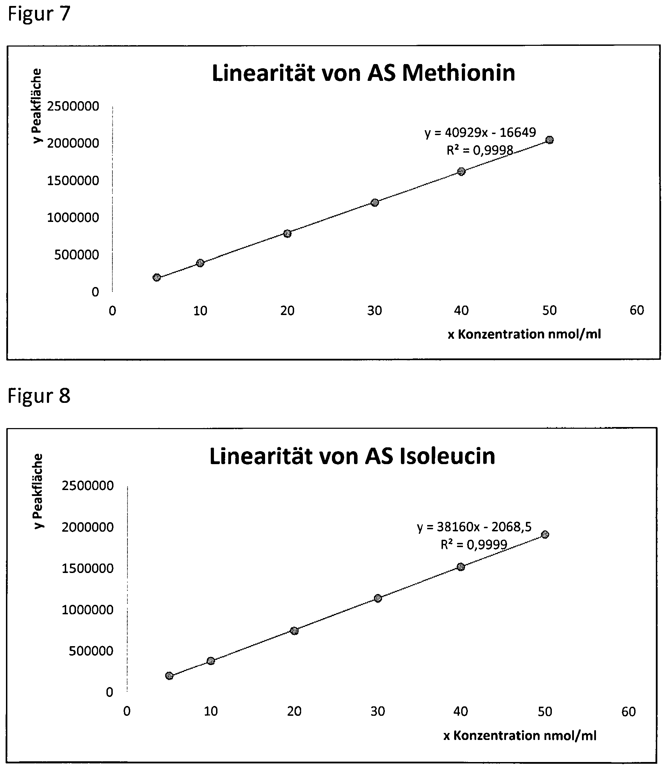

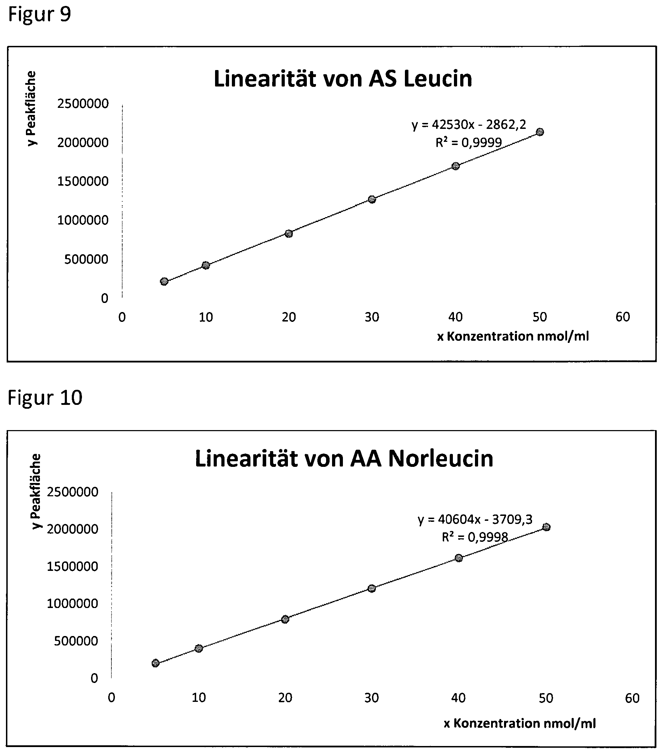

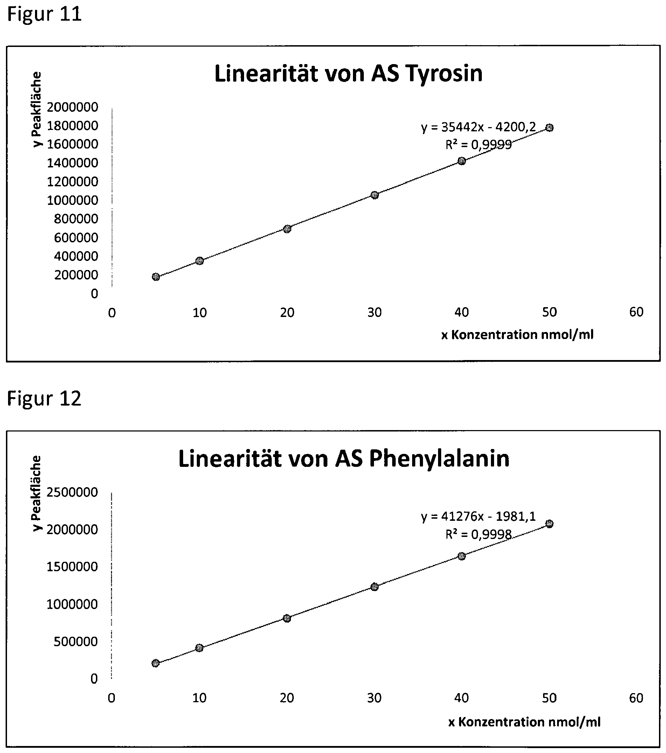

Nach der Trennung reagieren die Aminosäuren mit der Reagenzlösung Ninhydrin zu einem blauen Farbstoff (Ruhemanns Purpur). Die Intensität der Farbe ist der Konzentration des Farbstoffes und damit der Konzentration einer zu bestimmenden Aminosäure proportional. Zur quantitativen Analyse werden die Aminosäuren photometrisch bei 570 nm bestimmt und die Probe Standard mit bekannten Konzentrationen mit der zu untersuchenden Probe verglichen und ausgewertet (Figuren 1 und 2).

**Tabelle 1 Verlauf der Säulentemperatur**

| Stufe | von [h:min:sec] | bis [h:min:sec] | Temperatur 1 [°C] | Temperatur 2 [°C] |
|---|---|---|---|---|
| 1. | 00:00:00 | 00:34:28 | 57 | 57 |
| 2. | 00:34:28 | 00:37:02 | 57 | 70 |
| 3. | 00:37:02 | 00:46:18 | 70 | 70 |
| 4. | 00:46:18 | 00:50:21 | 70 | 57 |
| 5. | 00:50:21 | 00:55:03 | 57 | 57 |

pH Werte Eluenten:
B: 3,48 ± 0,2
C: 4,41 ± 0,2
F: 12,43 ± 0,2

### Methodenvalidierung

### Präzision

Durch die Ermittlung der Präzision wird nachgewiesen, dass die jeweiligen Messwerte unter den spezifischen Bedingungen der Analyse reproduzierbar sind. Prinzipiell unterscheidet man zwei Arten der Präzision: die intra- und die inter-assay Präzision. Bei der intra-assay Präzision werden die Konzentrationen von aufeinanderfolgenden Messungen (n = 10) des gleichen Plasmas innerhalb einer Versuchsreihe gemessen. Die erhaltenen Ergebnisse der einzelnen Messungen sollten möglichst nah beieinander liegen. Um die Inter-Assay Präzision zu ermitteln, wird die Präzision in verschiedenen Läufen (n = 10) und an verschiedenen Tagen mit demselben Plasma unter den gleichen Bedingungen bestimmt. Auch in diesem Fall wird erwartet, dass die Ergebnisse nah beieinander liegen. Als Maß für die Präzision gilt der Variationskoeffizient (VK) als Ausdruck für die Intra- und Interassay-Abweichungen. Aus den ermittelten und in Tabelle 2 und 3 dargestellten Ergebnissen lässt sich eine gute Intra-und Inter-assay-Reproduzierbarkeit der Methode ableiten.

**Tabelle 2 Intra-assay Präzision von AS H-Cys,Val, Met,Ile,Leu,Nle,Tyr und Phe**

| Messungen n=10 AS | Mittelwert | SD | VK (%) |
|---|---|---|---|
| H-Cys | *x̅* = 7,34 | ± 0,305 | 4,15 |
| Val | *x̅* = 164,4 | ± 0,855 | 0,52 |
| Met | *x̅* = 20,87 | ± 0,244 | 1,17 |
| lle | *x̅* = 46,22 | ± 0,269 | 0,58 |
| Leu | *x̅* = 107,2 | ± 0,586 | 0,55 |
| Nle | *x̅* = 99,53 | ± 0,607 | 0,61 |
| Tyr | *x̅* = 67,8 | ± 0,380 | 0,56 |
| Phe | *x̅* = 69,61 | ± 0,511 | 0,73 |

**Tabelle 3 Inter-assay Präzision von AS H-Cys, Val, Met,Ile,Leu,Nle,Tyr und Phe**

| Messungen n=10 AS | Mittelwert | SD | VK (%) |
|---|---|---|---|
| H-Cys | *x̅* = 7,56 | ± 0,436 | 5,77 |
| Val | *x̅* = 163,8 | ± 2,172 | 1,33 |
| Met | *x̅* = 21,24 | ± 0,592 | 2,76 |
| lle | x = 44,67 | ± 0,908 | 2,03 |
| Leu | *x̅* = 105,3 | ± 1,721 | 1,63 |
| Nle | *x̅* = 100,37 | ± 1,141 | 1,14 |
| Tyr | *x̅* = 69,13 | ± 0,830 | 1,2 |
| Phe | *x̅* = 70,35 | ± 0,617 | 0,88 |

### Wiederfindung

Für den Test der Wiederfindungsrate wurde eine Verdünnungsreihe hergestellt. Hierfür wurde eine Plasmaprobe mit einer Homocysteinlösung (Konzentration 40 nmol/ml) gespikt.

Plasmavorbereitung für die Verdünnungsreihe: 2 ml Plasma plus 2 ml Homocysteinlösung (Homocysteinkonzentration 40 nmol /ml).

**Tabelle 4 Schema Verdünnungsreihe**

| | | | | | | |
|---|---|---|---|---|---|---|
| Relative Konzentration des Plasmas | 100% | 80% | 60% | 40% | 20% | 10% |
| Plasmavolumen Zusatz | 1000 µl | 800 µl | 600 µl | 400 µl | 200 µl | 100 µl |
| Verdünnungspuffer Volumen | | 200 µl | 400 µl | 600 µl | 800 µl | 900 µl |

**Tabelle 5 Wiederfindungsrate % (gespiked mit 40 nmol H-Cys) Homocysteinkonzentration in nmol / ml Plasma**

| Konzentration | 100% | 80% | Wiederfindungsrate % | 60% | Wiederfindungsrate % | |
|---|---|---|---|---|---|---|
| Messungen n= 10 | | | | | | |
| Mittelwert | *x̅* = 43,48 | *x̅* = 35,21 | *x̅* = 80,98 | *x̅* = 26,0 | *x̅* = 59,8 | |
| Standardab weichung | SD ± 0,308 | SD ± 0,267 | SD + 0,666 | SD ±0,175 | SD ± 0,758 | |
| Variationskoeffizient | VK 0,71% | VK 0,76% | VK 0,82% | VK 0,67% | VK 1,27% | |

| Konzentration | 40% | Wiederfindungsrate % | 20% | Wiederfindungsrate % | 10% | Wiederfindungsrate % |
|---|---|---|---|---|---|---|
| Messungen n= 10 | | | | | | |
| Mittelwert | *x̅* = 17,15 | *x̅* = 39,43 | *x̅* = 8,77 | *x̅* = 20,23 | *x̅* = 4,26 | *x̅* = 9,79 |
| Standardab weichung | SD± 0,494 | SD± 1,092 | SD± 0,0898 | SD± 0,347 | SD± 0,0975 | SD ±0,197 |
| Variationskoeffizient | VK 2,88% | VK 2,77% | VK 1,02% | VK 1,71% | VK 2,29% | VK 2,01% |

**Tabelle 6 Wiederfindungsrate % Valinkonzentration in nmol / ml Plasma**

| Konzentration | 100% | 80% | Wiederfindungsrate % | 60% | Wiederfindungsrate % | |
|---|---|---|---|---|---|---|
| Messungen n= 10 | | | | | | |
| Mittelwert | *x̅* = 166,8 | *x̅* = 132,8 | *x̅* = 79,62 | *x̅* = 103,7 | *x̅* = 62,16 | |
| Standardabweichung | SD ± 0,739 | SD ± 0,83 | SD ± 0,4956 | SD ± 0,453 | SD ± 0,461 | |
| Variationskoeffizient | VK 0,44% | VK 0,63% | VK 0,62% | VK 0,44% | VK 0,74% | |
| | | | | | | |

| Konzentration | 40% | Wiederfindungsrate % | 20% | Wiederfindungsrate % | 10% | Wiederfindungsrate % |
|---|---|---|---|---|---|---|
| Messungen n= 10 | | | | | | |
| Mittelwert | *x̅* = 67,43 | *x̅* = 40,43 | *x̅* = 32,39 | *x̅* = 20,62 | *x̅* = 16,72 | *x̅* = 10,02 |
| Standardabweichung | SD± 0,256 | SD± 0,242 | SD ±0,12 | SD± 0,125 | SD ±0,148 | SD ± 0,087 |
| Variationskoeffizient | VK 0,38% | VK 0,60% | VK 0,37% | VK 0,58% | VK 0,89% | VK 0,87% |

**Tabelle 7 Wiederfindungsrate % Methioninkonzentration in nmol / ml Plasma**

| Konzentration | 100% | 80% | Wiederfindungsrate % | 60% | Wiederfindungsrate % | |
|---|---|---|---|---|---|---|
| Messungen n = 10 | | | | | | |
| Mittelwert | *x̅* = 21,99 | *x̅* = 17,4 | *x̅* = 79,12 | *x̅* = 13,62 | *x̅* = 61,94 | |
| Standardabweichung | SD± 0,154 | SD± 0,201 | SD± 1,076 | SD± 0,149 | SD ± 0,991 | |
| Variationskoeffizient | VK 0,70% | VK 1,16% | VK 1,36% | VK 0,1,09% | VK 2,29% | |
| | | | | | | |

| Konzentration | 40% | Wiederfindungsrate % | 20% | Wiederfindungsrate % | 10% | Wiederfindungsrate % |
|---|---|---|---|---|---|---|
| Messungen n = 10 | | | | | | |
| Mittelwert | *x̅* = 8,95 | *x̅* = 40,7 | *x̅* = 4,54 | *x̅* = 20,65 | *x̅* = 2,21 | *x̅* = 10,05 |
| Standardabweichung | SD± 0,037 | SD± 0,299 | SD ± 0,051 | SD± 0,275 | SD ± 0,272 | SD ±0,129 |
| Variationskoeffizient | VK 0,41% | VK 0,73% | VK 1,12% | VK 1,33% | VK 1,23% | VK 1,28% |

**Tabelle 8 Wiederfindungsrate % Isoleucinkonzentration in nmol / ml Plasma**

| Konzentration | 100% | 80% | Wiederfindungsrate % | 60% | Wiederfindungsrate % | |
|---|---|---|---|---|---|---|
| Messungen n = 10 | | | | | | |
| Mittelwert | *x̅* =44,75 | *x̅* = 35,55 | *x̅* = 79,44 | *x̅* = 27,84 | *x̅* = 62,21 | |
| Standardabweichung | SD± 0,213 | SD± 0,228 | SD± 0,463 | SD± 0,276 | SD + 0,893 | |
| Variationskoeffizient | VK 0,48% | VK 0,64% | VK 0,58% | VK 0,99% | VK 1,44% | |
| | | | | | | |

| Konzentration | 40% | Wiederfindungsrate % | 20% | Wiederfindungsrate % | 10% | Wiederfindungsrate % |
|---|---|---|---|---|---|---|
| Messungen n = 10 | | | | | | |
| Mittelwert | *x̅* = 18,40 | *x̅* = 41,12 | *x̅* = 9,50 | *x̅* = 21,23 | *x̅* = 4,79 | *x̅* = 10,71 |
| Standardabweichung | SD± 0,088 | SD± 0,157 | SD ± 0,085 | SD± 0,183 | SD ± 0,084 | SD ±0,183 |
| Variationskoeffizient | VK 0,48% | VK 0,8% | VK 0,89% | VK 0,86% | VK 1,75% | VK 1,71% |

**Tabelle 9 Wiederfindungsrate % Leucinkonzentration in nmol / ml Plasma**

| Konzentration | 100% | 80% | Wiederfindungsrate % | 60% | Wiederfindungsrate % | |
|---|---|---|---|---|---|---|
| Messungen n = 10 | | | | | | |
| Mittelwert | *x̅* = 109,3 | *x̅* = 87,36 | *x̅* = 79,54 | *x̅* = 68,06 | *x̅* = 61,97 | |
| Standardabweichung | SD± 0,591 | SD ± 0,459 | SD ± 0,376 | SD ± 0,642 | SD ± 0,857 | |
| Variationskoeffizient | VK 0,54% | VK 0,53% | VK 0,47% | VK 0,94% | VK 1,38% | |
| | | | | | | |

| Konzentration | 40% | Wiederfindungsrate % | 20% | Wiederfindungsrate % | 10% | Wiederfindungsrate % |
|---|---|---|---|---|---|---|
| Messungen n = 10 | | | | | | |
| Mittelwert | *x̅* = 44,27 | *x̅* = 40,31 | *x̅* = 22,56 | *x̅*=20,543 | *x̅* = 11,05 | *x̅*= 10,06 |
| Standardabweichung | SD± 0,196 | SD± 0,211 | SD ±0,199 | SD± 0,186 | SD + 0,109 | SD ± 0,086 |
| Variationskoeffizient | VK 0,44% | VK 0,52% | VK 0,88% | VK 0,91% | VK 0,98% | |

**Tabelle 10 Wiederfindungsrate % Tyrosinkonzentration in nmol / ml Plasma**

| Konzentration | 100% | 80% | Wiederfindungsrate % | 60% | Wiederfindungsrate % | |
|---|---|---|---|---|---|---|
| Messungen n = 10 | | | | | | |
| Mittelwert | *x̅* =69,89 | *x̅* = 54,71 | *x̅* = 78,28 | *x̅* = 43,44 | *x̅* = 62,15 | |
| Standardabweichung | SD± 0,412 | SD± 0,346 | SD± 0,449 | SD± 0,442 | SD ± 0,872 | |
| Variationskoeffizient | VK0,59 % | VK 0,63% | VK 0,57% | VK 1,02% | VK 1,40% | |
| | | | | | | |

| Konzentration | 40% | Wiederfindungsrate % | 20% | Wiederfindungsrate % | 10% | Wiederfindungsrate % |
|---|---|---|---|---|---|---|
| Messungen n = 10 | | | | | | |
| Mittelwert | *x̅* = 28,87 | *x̅* = 41,31 | *x̅* = 14,81 | *x̅* = 21,19 | x = 7,17 | *x̅* = 10,26 |
| Standardabweichung | SD± 0,154 | SD± 0,260 | SD ± 0,223 | SD± 0,344 | SD ±0,145 | SD ±0,181 |
| Variationskoeffizient | VK 0,53% | VK 0,63% | VK 1,51% | VK 1,62% | VK 2,02% | VK 1,76% |

**Tabelle 11 Wiederfindungsrate % Phenylalaninkonzentration in nmol / ml Plasma**

| Konzentration | 100% | 80% | Wiederfindungsrate % | 60% | Wiederfindungsrate % | |
|---|---|---|---|---|---|---|
| Messungen n = 10 | | | | | | |
| Mittelwert | *x̅* =69,12 | *x̅* = 55,12 | *x̅* = 79,74 | *x̅* = 43,09 | *x̅* = 62,34 | |
| Standardabweichung | SD± 0,474 | SD± 0,382 | SD± 0,596 | SD± 0,438 | SD ± 0,898 | |
| Variationsko effizient | VK 0,69% | VK 0,69% | VK 0,75% | VK 1,02% | VK 1,44% | |
| | | | | | | |

| Konzentration | 40% | Wiederfindungsrate % | 20% | Wiederfindungsrate % | 10% | Wiederfindungsrate % |
|---|---|---|---|---|---|---|
| Messungen n = 10 | | | | | | |
| Mittelwert | *x̅* = 28,79 | x= 41,65 | *x̅* = 14,79 | *x̅* = 21,40 | *x̅* = 7,76 | *x̅* = 11,23 |
| Standardabweichung | SD± 0,152 | SD± 0,269 | SD ±0,151 | SD± 0,295 | SD ±0,125 | SD ±0,174 |
| Variationskoeffizient | VK 0,53% | VK 0,65% | VK 1,02% | VK 1,38% | VK 1,61% | VK 1,55% |

### Linearität

Die Linearität gibt Aufschluss darüber, in welchem Konzentrationsbereich der geprüften Substanzen die Substanzmenge mit der Größe der Peakfläche linear ist. Die Linearität der verwendeten Substanzen im gemessenen Bereich wurde anhand von Eichgeraden überprüft. Die Eichgeraden wurden mit Excel erstellt und sind in nmol/ml dargestellt.. Zur Erstellung der Standardgeraden wurden je 20 µl der 6 Kalibrierlösungen (Konzentrationsbereich von 5 bis 50 nmol/ml) jeweils fünfmal injiziert. Bei der Berechnung wurden die Mittelwerte der Peakflächen gegen die Konzentrationen aufgetragen. Die Kalibrierkurven wurden als Trendlinien (Typ: linear) berechnet. Die Eichgeraden verliefen linear und es er gaben sich lineare Regressionen mit hohen Regressionskoeffizienten (siehe Tabelle 12).

**Tabelle 12**

| Peak Fläche Konzentration in nmol/ml | H-Cys | Val | Met | Ile | Leu | Nle | Tyr | Phe |
|---|---|---|---|---|---|---|---|---|
| 5 | 18183 | 75526 | 199761 | 197030 | 220565 | 209566 | 181370 | 211023 |
| 10 | 38216 | 145413 | 395214 | 381723 | 423615 | 404574 | 351586 | 415888 |
| 20 | 88123 | 287373 | 786096 | 748435 | 834465 | 794475 | 692616 | 812553 |
| 30 | 144053 | 434778 | 1203869 | 1139950 | 1267085 | 1207576 | 1053691 | 1232059 |
| 40 | 197942 | 582370 | 1619502 | 1521800 | 1696965 | 1620715 | 1417195 | 1640350 |
| 50 | 256779 | 737247 | 2039672 | 1913399 | 2132358 | 2034479 | 1771845 | 2074089 |
| Korrelationskoeffizient | 0,9983 | 0,9997 | 0,9998 | 0,9999 | 0,9999 | 0,9998 | 0,9999 | 0,9998 |

### Nachweisgrenze (NG) und Bestimmungsgrenze (BG)

Eine Methode beruht auf der Bestimmung der sogenannten Blindwerte bzw. Leerwerte. Dazu wurde eine Blindprobe (Verdünnungspuffer) vorbereitet und zehnmal gemessen, Mittelwert und Standardabweichung bestimmt. Zur Festsetzung der unteren Nachweisgrenze wurde die dreifache Standardabweichung festgelegt, wenn x mit einer 99,9% Wahrscheinlichkeit in den Nullbereich liegt, wenn nicht addiert man den mittleren Blindwert dazu.

Die Bestimmungsgrenze ist die kleinste Konzentration eines Analyten, die quantitativ mit einer festgelegten Präzision bestimmt werden kann. Erst oberhalb der Bestimmungsgrenze werden quantitative Analysenergebnisse angegeben. Die Bestimmungsgrenze hat immer eine höhere Genauigkeit als die Nachweisgrenze. Wie bei der Nachweisgrenze wird das Kriterium der gewünschten Genauigkeit in der Regel bezogen auf die Präzision des Blindwertes, d.h. auf den statistischen Fehler (ausgedrückt durch die Standardabweichung) des Messverfahrens bei einer Nullmessung oder Leermessung. Ein Messwert gilt häufig als quantitativ (bestimmt), wenn die Genauigkeit der Messung um den Faktor drei höher (besser) ist als die Genauigkeit der Nachweisgrenze, annähert dazu entspricht die Bestimmungsgrenze dann dem dreifachen Wert der Nachweisgrenze.

Quellen:
DIN 32645: Chemische Analytik; Nachweis-, Erfassungs- und Bestimmungsgrenze;
Ermittlung unter Wiederholbedingungen; Begriffe, Verfahren, Auswertung

**Tabelle 13 Nachweisgrenze (NWG) und Bestimmungsgrenze (BG)**

| AS | NWG (nmol/ml) | BG (nmol/ml) |
|---|---|---|
| H-Cys | 0,82 | 2,46 |
| Val | 0,74 | 2,22 |
| Met | 0,84 | 2,52 |
| Ile | 0,83 | 2,49 |
| Leu | 0,76 | 2,28 |
| Nie | 0,8 | 2,4 |
| Tyr | 0,76 | 2,28 |
| Phe | 0,89 | 2,27 |

**Tabelle 14**

| | | | | |
|---|---|---|---|---|
| Aminosäurekonzentration von Valin, Methionin, Isoleucin, Leucin, Tyrosin, and Phenylalanin in nmol/ml Blutplasma A ohne Reduktion und B mit Reduktion | | | | |

| | | A | | B |
|---|---|---|---|---|
| | *x̅* | SD | *x̅* | SD |
| AS | | | | |
| Val | 204,3 | ± 74,9 | 203,8 | ± 79,8 |
| Met | 35,8 | ± 12,4 | 35,2 | ± 11,8 |
| Ile | 77,5 | ± 33,3 | 77,8 | ± 35,8 |
| Leu | 126,8 | ± 46,6 | 125,7 | ± 48,5 |
| Tyr | 81,8 | ± 24,5 | 80,6 | ± 23,3 |
| Phe | 72,1 | ± 18,0 | 72,7 | ± 17,7 |

Werte sind Mittelwerte ± Standardabweichung (n = 10)

Die Reduktion von gebundenen Homocystein in freies Gasamthomocystein mit 4%-igen Dithiothreitollösung hat keinen signifikanten Einfluss auf die anderen Aminosäurekonzentrationen (Valin, Methionin, Ileucin, Leucin Tyrosin und Phenylalanin, Tabelle 14).

Die in den folgenden Tabellen gezeigten Beispiele sollen die Erfindung näher erläutern. Hierfür wurden Blutplasmaproben von gesunden Neugeborenen, Neugeborenen mit Ahornsiruperkrankung, Patienten ohne und mit Herzinfarkt, Patienten ohne und mit Diabetes mellitus Typ 2, wie vorher beschrieben aufbereitet und die Aminosäurekonzentrationen bestimmt.

**Tabelle 15**

| | | | | |
|---|---|---|---|---|
| Aminosäurekonzentration von Valin, Homocystein, Methionin, allo-lsoleucin, Isoleucin, Leucin, Tyrosin, und Phenylalanin in nmol/ml Blutplasma A Neugeborene ohne Ahornsirupkrankheit und B Neugeborene mit Ahornsirupkrankheit | | | | |

| | | A | | B |
|---|---|---|---|---|
| | *x̅* | SD | *x̅* | SD |
| AS | | | | |
| Val | 202,1 <a> | ± 54,1 | 467,9<b> | ± 116,9 |
| H-Cystein | 4,4<a> | ± 1,5 | 6,9<b> | ± 2,8 |
| Met | 31,1<a> | ± 7,9 | 17,2<b> | ± 4,0 |
| allo-Ile | <2<a> | | 66,2<b> | ± 37,4 |
| He | 58,9<a> | ± 16,9 | 213,0<b> | ± 48,6 |
| Leu | 141,9<a> | ± 40,5 | 433,6<b> | ± 108,1 |
| Tyr | 108,0<8> | ± 31,2 | 67,4<b> | ± 20,9 |
| Phe | 99,4<a> | ± 18,6 | 70,2<b> | ± 11,2 |

Werte sind Mittelwerte ± Standardabweichung (n = 10)
Mittelwerte mit unterschiedlichen Hochbuchstaben unterscheiden sich signifikant voneinander p ≤ 0,05

Bei Neugeborenen mit Ahornsirupkrankheit sind die Aminosäurekonzentrationen von Valin, Homocystein, allo-Isoleucin, Isoleucin und Leucin signifikant erhöht und die Aminosäurekonzentrationen von Methionin, Tyrosin und Phenylalanin signifikant niedriger im Vergleich zu den Neugeborenen ohne Ahornsirupkrankheit (Tabelle 15).

Vergleichende Studien: Oglesbee D. et. al. Second-Tier Test for Quantification of Alloisoleucine and Branched-Chain Amino Acids in Dried Blood Spots to Improve Newborn Screening for Maple Syrup Urine Disease (MSUD) Clinical Chemistry 54, No.:3 2008. Schadewaldt P. et al Significance of L-allo-isoleucine in plasma for diagnosis of maple syrup urine disease. Clin Chem. 1999 Oct;45(10): 1734-40. Refsum H. et. al. Screening for Serum Total Homocysteine in Newborn Children. Clinical Chemistry 50, No. 10, 2004

**Tabelle 16**

| | | | | |
|---|---|---|---|---|
| Aminosäurekonzentration von Valin, Homocystein, Methionin, allo-lsoleucin, Isoleucin, Leucin, Tyrosin, und Phenylalanin in nmol/ml Blutplasma A Patienten ohne Diabetes Typ 2 B Patienten mit Diabetes Typ 2 | | | | |

| | | A | | B |
|---|---|---|---|---|
| | *x̅* | SD | *x̅* | SD |
| AS | | | | |
| Val | 197,6 | ± 28,7 | 218,6 | ± 24,3 |
| H-Cystein | 10,7<a> | ± 4,0 | 19,6<b> | ± 2,6 |
| Met | 22,9 | ± 5,2 | 19,1 | ± 2,1 |
| Ile | 66,3 | ± 9,4 | 73,4 | ± 13,2 |
| Leu | 124,1 | ± 25,2 | 143,5 | ± 20,6 |
| Tyr | 65,5 | ± 5,4 | 74,9 | ± 15,1 |
| Phe | 101,4<a> | ± 21,8 | 56,9<b> | ± 7,2 |

Werte sind Mittelwerte ± Standardabweichung (n = 10)
Mittelwerte mit unterschiedlichen Hochbuchstaben unterscheiden sich signifikant voneinander p ≤ 0,05

Patienten mit Diabetes Typ 2 zeigten signifikant eine höhere Aminosäure-konzentration von Homocystein und eine signifikant niedrigere Aminosäure-konzentration von Phenylalanin im Vergleich zu Patienten ohne Diabetes Typ 2 Tabelle 16).

Vergleichende Studien: Buysschaert M. et.al.: Hyperhomocysteinemia in Type 2 Diabetes Diabetes Care, Volume 23, Number 12, December 2000. Singh M. A. et. al. Serum homocysteine in type 2 diabetes with cardiovascular complications J Med Soc 2013;27: 31-5.

**Tabelle 17**

| | | | | |
|---|---|---|---|---|
| Aminosäurekonzentration von Valin, Homocystein, Methionin, Isoleucin, Leucin, Tyrosin, and Phenylalanin in nmol/ml Blutplasma A Patienten ohne Herzinfarkt und B Patienten mit Herzinfarkt | | | | |

| | | A | | B |
|---|---|---|---|---|
| | *x̅* | SD | *x̅* | SD |
| AS | | | | |
| Val | 197,6<a> | ± 28,7 | 250,4<b> | ± 48,5 |
| H-Cystein | 10,7<a> | ± 4,0 | 29,7<b> | ± 4,2 |
| Met | 22,9 | ± 5,2 | 26,7 | ± 7,3 |
| Ile | 66,3<a> | ± 9,4 | 88,4<b> | ± 12,4 |
| Leu | 124,1 <a> | ± 25,2 | 159,1<b> | ± 23,7 |
| Tyr | 65,5<a> | ± 5,4 | 83,5<b> | ± 21,9 |
| Phe | 101,4 | ± 21,8 | 102,6 | ± 27,4 |

Werte sind Mittelwerte ± Standardabweichung (n = 10)
Mittelwerte mit unterschiedlichen Hochbuchstaben unterscheiden sich signifikant voneinander p ≤ 0,05

Bei Patienten mit Herzinfarkt sind die Aminosäurekonzentrationen von Homocystein, Valin, Isoleucin, Leucin und Tyrosin signifikant erhöht im Vergleich zu Patienten ohne Herzinfarkt (Tabelle 17).

Vergleichende Studien: Nygard O. et.al. Plasma Homocysteine Levels and Mortality in Patients with coronary artery disease. The New England Journal of Medicine Volume 337 Number 4 July 24, 1997. Wali V. et. al. Serum Homocysteine Levels As a Novel Biomarker in Patients with Acute Myocardial Infarction. International Journal of Medical and Health Sciences October 2012,Vol-1; Issue-4.

### Schlussfolgerung

Die entwickelte Methode zur Bestimmung der Aminosäuren Homocystein, Valin, Methionin, Isoleucin, Leucin, Tyrosin und Phenylalanin im Blutplasma ist linear, präzise und reproduzierbar.

Der Test ist kostengünstig, leicht in der Handhabung und Zeit einsparend.

Einsatz der Methode im Neugeborenen-Screening zur Früherkennung von angeborenen Stoffwechselerkrankungen, wie Phenylketonurie (PKU), Ahornsirupkrankheit (MSUD) und Hyperhomocysteinämie. Der Vorteil der Methode ist die kombinierte bzw. gleichzeitige Bestimmung von den Aminosäure-konzentrationen Homocystein, Valin, Methionin, Isoleucin, Leucin, Tyrosin und Phenylalanin im Blutplasma, und ein zweiter Test für die Bestimmung von Homocystein ist nicht mehr notwendig, somit werden zusätzlich Arbeitszeit und Kosten eingespart.

Anwendung der Methode in medizinischen Laboratorien, insbesondere in der Klinischen Chemie für diagnostische Zwecke zur Früherkennung und Prävention von Erkrankungen, insbesondere Herz-Kreislauferkrankungen und Diabetes Typ 2 bei Risikopatienten.

Dem Patienten soll eine zielgerechte Beurteilung über seine Gesundheit und deren Zustand gegeben werden, was wiederum zu einer Vermeidung bzw. Verhütung von voraussehbaren Erkrankungen führt und einen positiven Einfluss auf den Einsatz von gesundheitsfördernden Maßnahmen hat und letztlich zum Erfolg führen.

Die Gesamtkosten von Patienten mit Herz-Kreislauf-Erkrankungen und Diabetes Mellitus Typ 2 sind in den letzten Jahren auf ca. 56 Mrd. Euro jährlich gestiegen.

Daher ist die Einführung eines diagnostischen Tests zur Früherkennung von Herz-Kreislauf-Erkrankungen und Diabetes Mellitus Typ 2 nicht nur aus medizinischer Sicht, sondern auch ökonomisch von Vorteil. Einmal wird die Lebensqualität verbessert, und zum anderen verringern sich die Behandlungskosten von den Folgeerkrankungen.

## Patentansprüche

1. Verfahren zur chromatografischen quantitativen Bestimmung der Aminosäuren Valin, Methionin, allo-Isoleucin, Isoleucin, Leucin, Tyrosin und Phenylalanin im Blutplasma, wobei die Aminosäure Homocystein in einem einzigen Chromatografielauf quantitativ miterfasst wird, wobei es sich beim Verfahren um ein mehrere Eluenten und ein Trennprogramm verwendendes chromatografisches Verfahren handelt,
**dadurch gekennzeichnet,**
**dass** es sich bei den Eluenten um folgende handelt, mit jeweils folgender relativer Zusammensetzung:
B: Lithiumhydroxid-Monohydrat 2,5 g, Borsäure 0,5 g, Ethanol 5 ml, Caprylsäure 0,1 ml, Trifluoressigsäure-Lösung 50% 4,7 ml, Reinstwasser 480 ml;
C: Lithiumhydroxid-Monohydrat 2,1 g, Borsäure 1,5 g, Ameisensäure 1,5 ml, Essigsäure 100% 1,5 ml, Caprylsäure 0,1 ml, Trifluoressigsäure-Lösung 50% 0,8 ml, Reinstwasser 495 ml.

2. Verfahren nach Anspruch 1, **dadurch gekennzeichnet, dass** die Eluenten bei den nachfolgenden pH-Werten verwendet werden:
Eluent B: pH-Wert entspricht 3,46;
Eluent C: pH-Wert entspricht 4,48.

3. Verfahren nach einem der Ansprüche 1 bis 2, **dadurch gekennzeichnet, dass** die Eluenten B bis C nacheinander in der Reihenfolge B, C verwendet werden.

4. Verfahren nach einem der Ansprüche 1 bis 3, **dadurch gekennzeichnet, dass** das folgende Trennprogramm verwendet wird, wobei die Daten sich auf eine Eluentenflussrate von 240 Mikroliter je Minute und folgenden Eluenten- und Säulentemperaturverlauf beziehen:
Eluent B: Zeit ab 0 Stunden, 0 Minuten bis 9 Minuten, 57 Sekunden;
Eluent C: Zeit ab 9 Minuten, 57 Sekunden bis 40 Minuten, 3 Sekunden;
Säul entemperaturverlauf:
Zeit: 0 Stunden, 0 Minuten bis 34 Minuten, 28 Sekunden;
Säulentemperatur: +57 Grad Celsius bei 0 Stunden, 0 Minuten;
Säulentemperatur: +57 Grad Celsius bei 34 Minuten, 28 Sekunden;
Zeit: 34 Minuten, 28 Sekunden bis 37 Minuten, 2 Sekunden;
Säulentemperatur: +57 Grad Celsius bei 34 Minuten, 28 Sekunden;
Säulentemperatur: +70 Grad Celsius bei 37 Minuten, 2 Sekunden;
Zeit: 37 Minuten, 2 Sekunden bis 40 Minuten, 3 Sekunden;
Säulentemperatur: +70 Grad Celsius bei 37 Minuten, 2 Sekunden;
Säulentemperatur: +70 Grad Celsius bei 40 Minuten, 3 Sekunden,
wobei zwischen den jeweiligen zeitlichen Anfangs- und Endpunkten bei angegebenen Temperaturdifferenzen der Temperaturverlauf zwischen den unterschiedlichen Temperaturniveaus im Wesentlichen linear verläuft.

5. Verfahren nach einem der Ansprüche 1 bis 4, **dadurch gekennzeichnet, dass** eine Trennsäule verwendet wird, die gepackt ist mit Polymerharz aus Styrol und Divinylbenzol und einer Vernetzung von 10%.

6. Verfahren nach Anspruch 5, **dadurch gekennzeichnet, dass** die Abmessungen der Trennsäule 4 mm im Innendurchmesser und 118 mm Innenlänge und 125 mm Außenlänge sind.

7. Verwendung eines Eluenten nach Anspruch 8 in einem Verfahren nach einem der Ansprüche 1 bis 6.

8. Eluent, ausgewählt aus einer Gruppe, bestehend aus:
B: Lithiumhydroxid-Monohydrat 2,5 g, Borsäure 0,5 g, Ethanol 5 ml, Caprylsäure 0,1 ml, Trifluoressigsäure-Lösung 50% 4,7 ml, Reinstwasser 480 ml;
C: Lithiumhydroxid-Monohydrat 2,1 g, Borsäure 1,5 g, Ameisensäure 1,5 ml, Essigsäure 100% 1,5 ml, Caprylsäure 0,1 ml, Trifluoressigsäure-Lösung 50% 0,8 ml, Reinstwasser 495 ml,
wobei die Zusammensetzungsangaben der Bestandteile im relativen Sinne zu verstehen sind.

9. Eluent nach Anspruch 8, geeignet für eine Verwendung in einem Verfahren nach einem der Ansprüche 1 bis 6.

## Claims

1. Method for chromatographic quantitative determination of the amino acids valine, methionine, alloisoleucine, isoleucine, leucine, tyrosine and phenylalanine in the blood plasma, wherein the amino acid homocysteine is also recorded quantitatively in a single chromatography run, wherein the method is a chromatographic method using several eluents and a separating programme, **characterised in that** the eluents are the following, having respectively the following relative composition:
B: lithium hydroxide monohydrate 2.5 g, boric acid 0.5 g, ethanol 5 ml, caprylic acid 0.1 ml, trifluoroacetic acid solution 50% 4.7 ml, ultrapure water 480 ml;
C: lithium hydroxide monohydrate 2.1 g, boric acid 1.5 g, formic acid 1.5 ml, acetic acid 100% 1.5 ml, caprylic acid 0.1 ml, trifluoroacetic acid solution 50% 0.8 ml, ultrapure water 495 ml.

2. Method according to claim 1, **characterised in that** the eluents are used at the following pH values:
eluent B: pH value corresponds to 3.46;
eluent C: pH value corresponds to 4.48.

3. Method according to one of claims 1 to 2, **characterised in that** the eluents B to C are used one after another in the sequence B, C.

4. Method according to one of claims 1 to 3, **characterised in that** the following separating programme is used, wherein the data relate to an eluent flow rate of 240 microlitres per minute and the following eluent profile and column temperature profile:
eluent B: time from 0 hours, 0 minutes to 9 minutes, 57 seconds;
eluent C: time from 9 minutes, 57 seconds to 40 minutes, 3 seconds;
column temperature profile:
time: 0 hours, 0 minutes to 34 minutes, 28 seconds;
column temperature: + 57 degrees Celsius at 0 hours, 0 minutes;
column temperature: + 57 degrees Celsius at 34 minutes, 28 seconds;
time: 34 minutes, 28 seconds to 37 minutes, 2 seconds;
column temperature: + 57 degrees Celsius at 34 minutes, 28 seconds;
column temperature: + 70 degrees Celsius at 37 minutes, 2 seconds;
time: 37 minutes, 2 seconds to 40 minutes, 3 seconds;
column temperature: + 70 degrees Celsius at 37 minutes, 2 seconds;
column temperature: + 70 degrees Celsius at 40 minutes, 3 seconds,
wherein the temperature profile between the different temperature levels runs substantially linearly between the respective chronological initial and end points for indicated temperature differences.

5. Method according to one of claims 1 to 4, **characterised in that** a separating column is used which is packed with polymer resin made from styrene and divinylbenzene and crosslinking of 10%.

6. Method according to claim 5, **characterised in that** the dimensions of the separating column are 4 mm in internal diameter and 118 mm inner length and 125 mm outer length.

7. Use of an eluent according to claim 8 in a method according to one of claims 1 to 6.

8. Eluent selected from a group consisting of:
B: lithium hydroxide monohydrate 2.5 g, boric acid 0.5 g, ethanol 5 ml, caprylic acid 0.1 ml, trifluoroacetic acid solution 50% 4.7 ml, ultrapure water 480 ml;
C: lithium hydroxide monohydrate 2.1 g, boric acid 1.5 g, formic acid 1.5 ml,
acetic acid 100% 1.5 ml, caprylic acid 0.1 ml, trifluoroacetic acid solution 50% 0.8 ml, ultrapure water 495 ml,
wherein the composition details of the constituents are to be understood in the relative sense.

9. Eluent according to claim 8, suitable for use in a method according to one of claims 1 to 6.

## Revendications

1. Procédé de détermination quantitative chromatographique des acides aminés valine, méthionine, allo-isoleucine, isoleucine, leucine, tyrosine et phénylalanine dans le plasma sanguin, l'acide aminé homocystéine étant lui aussi quantitativement détecté dans une opération chromatographique unique, le procédé étant un procédé chromatographique utilisant plusieurs éluants et un programme de séparation,
**caractérisé en ce que**,
pour ce qui concerne les éluants, il s'agit des suivants, ayant chacun la composition relative suivante :
B : hydroxyde de lithium monohydraté 2,5 g, acide borique 0,5 g, éthanol 5 ml, acide caprylique 0,1 ml, solution d'acide trifluoracétique à 50 % 4,7 ml, eau ultra-pure 480 ml ;
C : hydroxyde de lithium monohydraté 2,1 g, acide borique 1,5 g, acide formique 1,5 ml, acide acétique à 100 % 1,5 ml, acide caprylique 0,1 ml, solution d'acide trifluoracétique à 50 % 0,8 ml, eau ultra-pure 495 ml.

2. Procédé selon la revendication 1, **caractérisé en ce que** les éluants sont utilisés aux pH suivants :
éluant B : le pH correspond à 3,46 ;
éluant C : le pH correspond à 4,48.

3. Procédé selon l'une des revendications 1 à 2, **caractérisé en ce que** les éluants B à C sont utilisés successivement dans l'ordre B, C.

4. Procédé selon l'une des revendications 1 à 3, **caractérisé en ce qu'**on utilise le programme de séparation suivant, les données se rapportant à un débit de l'éluant de 240 microlitres par minute, et aux programmes d'élution et de température de colonne suivants :
éluant B : temps 0 heure 0 minute à 9 minutes 57 secondes ;
éluant C : temps 9 minutes 57 secondes à 40 minutes 3 secondes ;
programme de température de colonne :
temps : 0 heure 0 minute à 34 minutes 28 secondes ;
température de colonne : + 57 degrés Celsius à 0 heure 0 minute ;
température de colonne : + 57 degrés Celsius à 34 minutes 28 secondes ;
temps : 34 minutes, 28 secondes à 37 minutes 2 secondes ;
température de colonne : + 57 degrés Celsius à 34 minutes 28 secondes ;
température de colonne : + 70 degrés Celsius à 37 minutes 2 secondes ;
temps : 37 minutes 2 secondes à 40 minutes 3 secondes ;
température de colonne : + 70 degrés Celsius à 37 minutes 2 secondes ;
température de colonne : + 70 degrés Celsius à 40 minutes 3 secondes,
l'évolution de la température, entre les différents niveaux de température,
s'effectuant pour l'essentiel d'une manière linéaire entre les points temporels de départ et de fin, pour les différences de température indiquées.

5. Procédé selon l'une des revendications 1 à 4, **caractérisé en ce qu'**on utilise une colonne de séparation qui est garnie d'une résine polymère de styrène et de divinylbenzène, avec un taux de réticulation de 10 %.

6. Procédé selon la revendication 5, **caractérisé en ce que** les dimensions de la colonne de séparation sont de 4 mm pour le diamètre intérieur et de 118 mm pour la longueur intérieure et de 125 mm pour la longueur extérieure.

7. Utilisation d'un éluant selon la revendication 8 dans un procédé selon l'une des revendications 1 à 6.

8. Eluant choisi dans un groupe consistant en :
B : hydroxyde de lithium monohydraté 2,5 g, acide borique 0,5 g, éthanol 5 ml, acide caprylique 0,1 ml, solution d'acide trifluoracétique à 50 % 4,7 ml, eau ultra-pure 480 ml ;
C : hydroxyde de lithium monohydraté 2,1 g, acide borique 1,5 g, acide formique 1,5 ml, acide acétique à 100 % 1,5 ml, acide caprylique 0,1 ml, solution d'acide trifluoracétique à 50 % 0,8 ml, eau ultra-pure 495 ml, les indications de composition des constituants s'entendant dans un sens relatif.

9. Eluant selon la revendication 8, convenant à une utilisation dans un procédé selon l'une des revendications 1 à 6.
